Europäisches Patentamt

European Patent Office

Office européen des brevets

(11) Publication number: **0 192 687**
**B1**

# EUROPEAN PATENT SPECIFICATION

(45) Date of publication of patent specification: 15.11.89

(51) Int. Cl.⁴: **A 41 B 13/02**, A 61 F 13/18

(21) Application number: **85904242.6**

(22) Date of filing: **21.08.85**

(86) International application number:
**PCT/DK85/00081**

(87) International publication number:
**WO 86/01378 13.03.86 Gazette 86/06**

(54) AN ABSORPTION BODY, NOTABLY FOR USE IN CASES OF URINARY INCONTINENCE IN WOMEN.

(30) Priority: **21.08.84 DK 3988/84**

(43) Date of publication of application:
**03.09.86 Bulletin 86/36**

(45) Publication of the grant of the patent:
**15.11.89 Bulletin 89/46**

(84) Designated Contracting States:
**AT BE CH DE FR GB IT LI LU NL SE**

(56) References cited:
**DE-A-2 319 309**
**SE-A- 222 269**
**SE-B- 348 239**
**SE-B- 362 357**
**SE-B- 433 430**
**US-A-3 343 543**
**US-A-3 670 731**

(73) Proprietor: **COLOPLAST A/S**
**4, Bronzevej**
**DK-3060 Espergaerde (DK)**

(72) Inventor: **KAMSTRUP-LARSEN, Jorgen**
**Kratbjergvej 10**
**DK-3450 Allerod (DK)**

(74) Representative: **Hartley, David et al**
**c/o Withers & Rogers 4 Dyer's Buildings Holborn**
**London, EC1N 2JT (GB)**

Courier Press, Leamington Spa, England.

## Description

Field of the Invention

The present invention relates to an absorption body notably meant for use in cases of urinary incontinence in women, but in principle also suitable for use as a diaper in other connections and as a dressing material for highly suppurating wounds and as an absorbing material for purulent matter and other liquids led away from, e.g., surgical operations. The absorption body according to the invention, which is of the kind that contains a core of a highly absorbent material, may also find use in some industrial connections, e.g. in chemical laboratories.

Background of the Invention

Many embodiments of diapers, sanitary napkins and catamenial tampons and similar absorption bodies are known. However, no known construction is particularly suitable for female urinary incontinence, which poses certain quite special demands. The absorption body must be flat, have a modest thickness in the dry condition and have a suitable width. It must be fairly uniform in its entire length and width, firstly so as to not require the exertion of a high degree of precision at its application and secondly so as to cause no harm if displaced while being worn by the user, such displacement being almost inevitable.

A particularly important requirement is that the absorption body is able to absorb very rapidly a not insignificant amount of urine. In cases of female urinary incontinence it frequently happens that in a vigorous flow (15-25 ml/sec.) urine flows out for, e.g., 1/2 to several seconds. These amounts of urine should be subject to absorption at once and that with a great certainty against overflow. A total absorption capacity in the absorption body of for instance 70 ml in a number of cases may be considered sufficient, but for some disorders it must be greater. An absorption body for the main purpose stated thus must have properties different from those of sanitary napkins which are to absorb a fluid flowing more constantly and slowly and having a much higher viscosity than urine.

It is known to manufacture absorption bodies with a receiving chamber from which urine may be distributed into the core of the absorption body. This has been found, however, to involve the drawback that the urine may have difficulties in being distributed into the entire core because the parts thereof bordering on the receiving chamber are rapidly saturated and thereby reduce the possibility of through-flow. Known sanitary napkins moreover have a tendency to press out the urine when they are compressed in a state entirely or partly filled with urine.

It will be understood that an absorption body for the present particular purpose must have a series of properties. One of them is that the abovementioned receiving chamber is replaced by a number of channels — preferably rectilinear and parallel — in the core body responsible for the absorption of the urine.

From DK patent specification No. 122,636 there is known a diaper of a highly absorbent material, preferably dry-fibered cellulose, having several depressions (which may be rectilinear and largely parallel). The depressions have been made by compression and this will be a considerable disadvantage in an absorption body for the use here contemplated because the compression will slow down somewhat the rate of the absorption of fluid and thereby render the diaper unsuitable for the absorption of such vigorous flow of urine as is usual in the case of incontinence.

From the German published specification No. 2 319 309, which is mainly concerned with the kind of the absorbing material (CMC-derivatives), it is known to provide a core with longitudinal, parallel depressions. It appears that they have been formed by embossing, i.e. compression, and thereby have the above-mentioned drawback in relation to an absorption body with the purpose here aimed at.

From US Patent specification No. 3 411 504 there is known a sanitary napkin which at the side facing the source of fluid is provided with a ridge situated between two grooves, the ridge being adapted to fit in between the labiae majorae in order to ensure against leakage. The grooves are preferably formed by compression, that method being stated to increase the absorption capacity. Such an absorbing object is not either suitable for the purpose aimed at with the present invention, i.a. because there are precisely only two grooves and the object therefore cannot absorb uniformly and evenly over its entire surface.

From U.S. 3,343,543 there is known a sanitary napkin provided with troughs for the absorption of liquid. These troughs normally have been formed by compression—with the above-mentioned drawbacks in relation to the use here contemplated—but it is stated that they may be formed by cutting although it has not been explained how or for which purpose. It is also stated in that specification that one or more layers of wrinkled or grooved paper may be present in the body of the napkin between its upper and lower faces to retard the flow of fluid to the bottom region thereof, which is stated to ensure total absorption by the upper region of the napkin before a material quantity of the fluid reaches and is absorbed by the lower portion. This measure shows that with this absorbing object one does not attempt at obtaining the fastest possible rate of the absorption of fluid, which presumably is connected with the higher viscosity of the material to be absorbed and perhaps also with the fact that it comes in a relatively slow flow.

## Brief Description of the Invention

It is the object of the invention to provide an absorption body that remedies the described drawback in the prior art and represents a combination of properties such that it is particularly suitable for use in cases of female urinary incontinence.

In one aspect the invention provides an absorption body for use in cases of urinary incontinence in women and containing a core of a highly absorbent material, said core optionally being entirely or partly surrounded by one or more layers of absorbing material, wherein:

a) the core is made up in the form of a flat pad and consists substantially of a fibrous material of which at least a part constitutes a stable, three-dimensional network as a structure-giving component in which there is evenly distributed highly absorbent material;

b) the core is provided with a number of preferably rectilinear and parallel cut channels on one side thereof;

characterised in that the core is provided with a thin, coherent porous layer of material on the side opposite said channels, said channels having a depth of 65 percent to 95 percent of the thickness of the pad, have a width of at least 4mm, and leave at least 4 mm width of core material between each two neighbouring channels, said fibrous material comprises thermoplastic fibres or fibres coated with thermoplastic material fused together at their points of contact and fused to said thin, coherent porous layer and said highly absorbent material is a superabsorbent material.

In another aspect the invention provides a method for producing an absorption body for use in cases of female urinary incontinence and containing a core in the form of a flat absorbent pad, and forming parallel channels in a major surface of the pad, characterised in that fibres, at least a proportion of which are formed of non-meltable thermoplastic material or are coated with thermoplastic materials are dry-deposited on a thin, coherent porous sheet together with pulverulent superabsorbent material, the sheet with the deposited layer of mixed fibrous material and pulverulent material is heated to a temperature sufficient to adhere the fibres together at their points of contact and to the sheet material, thereby forming three-dimensional coherent network in which the superabsorbent material is evenly distributed, and in that said channels are cut or milled to a depth of 65 percent to 95 percent of the thickness of the pad, each channel having a width of at least 4 mm and the spacing between neighbouring channels being at least 4 mm.

Preferred embodiments are defined in the dependent claims.

The principle of dry-depositing materials such as the core in question is well known. It consists in that the material from which the materials are to be prepared is deposited from an air-current on a suitable (optionally removable) support. Some degree of stratification is thereby achieved so that the material becomes somewhat anisotropic, yet not more so than it is justifiable to say that it has a uniform structure throughout the thickness. In the finished product this stratification ensures that by moistening and absorption of liquid the pad only expands in the height dimension whereas the width and length dimensions remain substantially unaffected. Such a stability of the flat shape of the absorption body not only is important for the comfort when it is worn but is also important for retaining the open cross-section of the channels after a first absorption of fluid so that the absorption body is ready for possibly absorbing later new liquid at approximately the same efficiency.

In preparation the portion of the fibrous material to form the structure-giving component in practice will be present as staple fibres or comparatively short lengths of filaments. This part of the content of the pad according to the invention preferably consists of fibres of a thermoplastic material; polyethylene fibres have proved particularly suitable but even polypropylene fibres are suitable. In principle all thermoplastic fibres may be used provided they can be hot-melted within a reasonable temperature range, e.g. 90-175°C. This fibrous material may also be natural fibres or other non-thermoplastic fibres coated with a thermoplastic material.

A thermoplastic surface is important. When these fibres have been deposited on the support and afterwards heated to a temperature just sufficient to soften the surfaces of the fibres, the fibres will adhere to each other at the spots of contact, whereby the three-dimensional network mentioned is formed. By the heat treatment also the thin, coherent layer or material contributing to the stiffening of the structure is adhered.

This network retains the other components of the pad. The most important one thereof is the highly absorbent material which is sometimes designated (and may be commercially available) with the name "superabsorber". Such materials may be either pulverulent or fibrous. As far as is known the first-mentioned form is the most common form of such materials and especially for retaining a powder the three-dimensional network is essential.

Several kinds of such materials are on the market. Some forms are based on cellulose or starch derivatives, others on cross-linked polymers of acrylic or methacrylic monomers. One kind of such a material that has proven very suitable for the present purpose is that sold under the name of "Favor" (a registered Trade Mark) and is supplied by Chemische Fabrik Stockhausen, Krefeld, BRD. This particular material can absorb up to many hundred times its own weight of distilled water. For urine the absorbing powder of various kinds of materials in question is 10 - 80 times their own weight and the absorbing capacity is not only high but also rapid. However, "Superabsorbers" exist with varying rates of absorption, which afford the possibility of obtaining an optimal effect. The amount of "superabsorber" is typically 1.5-5 g per absorption body. It is known to use these materials in connection with incontinence. The materials are insoluble but highly swellable in water.

The requirement of the method aspect of the invention that the channels have a depth of 65 - 95% of the thickness of the pad results in a considerable absorbing surface, viz. not only the face of the pad but also the walls of the channels. When urine flows from the urethra the major portion thereof will at once flow into the channels whereby it is avoided that any portion bypasses the absorption body and wets the legs or underwear of the wearer, and because of the big surface area the urine will immediately be absorbed from the channel walls and bottoms, that is if firstly the above-discussed condition (a) and second the condition (c) discussed hereinafter are fulfilled.

It is here mentioned that expediently the pad has a thickness of 2 - 7 mm, e.g. 3 - 6 mm and preferably about 5 mm.

The channels must be formed by a working of cutting or milling, not by compression or embossing. This has several advantages. First and foremost one hereby avoids the compression of the core material mentioned by way of introduction and which will delay the absorption of urine. But moreover it involves an economic advantage because the material cut away, notably the "superabsorber" thereof — which is the most expensive part of the absorption body according to the invention — then may be reused. The cutting must take place after the heat treatment by which the stable structure has been formed.

The channels must have a width of at least 4 mm in order to be able to absorb immediately a stream of urine and distribute it to the absorbing channel walls, and there must be at least 4 mm between two neighbouring channels so as to ensure a sufficient amount of material between the channels (plus at the bottoms of the channels) for the absorption. Expediently, the channels are about 5 mm wide and have an intermediary ridge having a width of about 7 mm.

It will be understood that a rather considerable amount of material is cut away or milled away from the pad after the dry-depositing, in practice normally about 30%, in some cases perhaps as low as about 25%. Because of the thin, coherent layer of material the core even after the removal of the large amounts of material does have, however, sufficient strength and rigidity to exhibit the appearance of a flat, flexible pad yet without tendency to fold up in the channels.

Description of the Drawing

In the following the invention will be described more fully with reference to the drawing in which:

Figure 1 shows a plan view of an absorption body according to the invention, the core being intimated by dashed lines; and

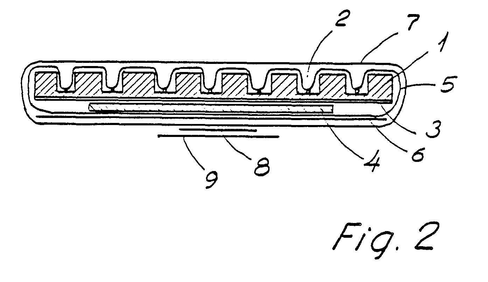

Figure 2 is a section of the absorption body along line II-II in Figure 1.

In the drawing the absorption body according to the invention is shown in a preferred embodiment particularly well suited for use in cases of female urinary incontinence. Inside the body there is a core 1 on which there has been placed various fluid distributing and absorbing layers, and outermost the absorption body is surrounded by a casing 7.

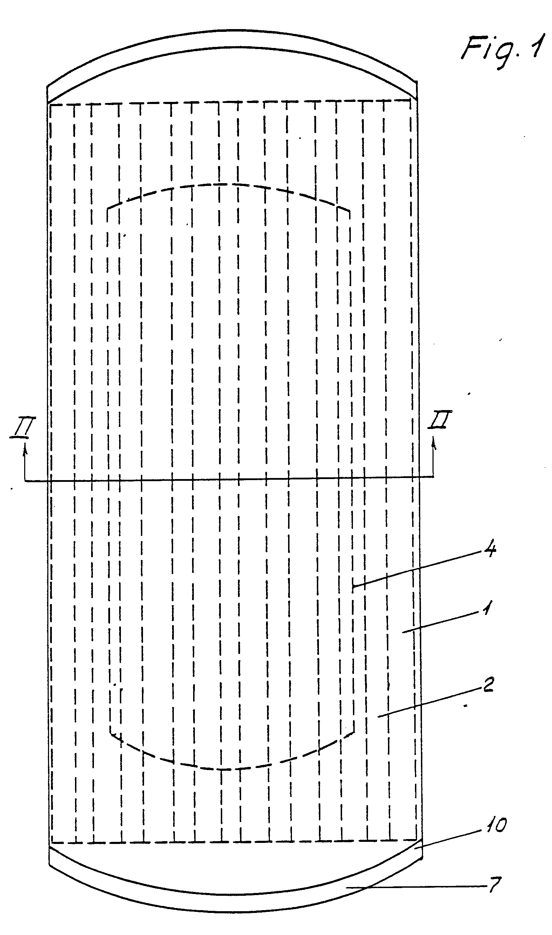

The core 1 has been prepared by dry-deposition in a continuous operation, and seen from above it has a rectangular shape as appears from Figure 1. The core may have a width of about 85 mm and a thickness of about 5 mm. The thickness may vary according to the desired absorption capacity and flexibility of the absorption body. The thickness will be between 2 and 12 mm, generally between 3 and 6 mm and preferably 5 mm. The material is normally constituted by a mixture of cellulose fluff, which is a filler having no small ability of absorbing fluids, in an amount of about 38% by weight, about 16% by weight of thermoplastic fibres and about 46% by weight of a so-called "superabsorber" which is a popular name of a class of highly absorbing materials. Such materials may be either pulverulent or fibrous and several kinds thereof are on the market. Some kind are based on cellulose or starch derivatives, others on cross-linked polymers of acrylic or methacrylic monomers. One suitable kind of such a material is sold under the mark "Favor" (registered trade mark) and is supplied by Chemische Fabrik Stockhausen, Krefeld, BRD. Optionally the content of cellulose fluff may be reduced or possibly entirely omitted but normally it is present in an amount as here relative the thermoplastic fibres of 2.5:1. The thermoplastic fibres are mandatory and may be polyethylene fibres, which are particularly suitable, but even for instance polypropylene fibres. In principle all kinds of thermoplastic fibres may be employed, only they can be hot-melted within a reasonable temperature range, e.g. 90-200°C. The upper limit of the temperature is normally set by the superabsorber, which often does not stand temperatures above 160°C. The fibrous material may even be natural fibres or other non-thermoplastic fibres coated with a thermoplastic material.

By the dry-deposition the material to enter the core is suspended in an air current and the material is deposited on a length of a thin, coherent, porous material, e.g. paper, the air being sucked off via the pores of the material. This mixture, constituting the core material, by the dry-deposition will be deposited in such a manner that the fibres primarily become oriented in planes extending approximately parallel to the upper and lower faces of the core. It is hereby obtained that the swelling at absorbing fluid into the core substantially takes place in the direction of the thickness of the core and thereby entails the least possible risk that the fluid will be pressed out by a local compression of the body.

The core is provided with straight and parallel channels or grooves 2 lengthwise of the upper face of the core. The forming of these channels or grooves 2 normally takes place after the body has been subjected to a heat treatment during which the thermoplastic fibres are heated at such high temperature (e.g. 90-200°C depending on the thermoplastic material) that in a portion of the points of intersection they fuse to form a three-dimensional lattice having a considerable stability of shape. Normally the superabsorber defines the upper limit of the temperature. The other components are evenly distributed within this lattice structure. This structure in connection with material length 3 melted onto it has a sufficient strength to allow the channels 2 to be formed by cutting or milling. The channels must have a width of at least 4 mm and there must be at least 4 mm between two neighbour channels. Ordinarily the channels are made at a width of 5 mm and with 7 mm width of the intermediary parts. The channel depth is 65-95% of the thickness of the core. The channels constitute distributing paths for fluid in the body, ensure a considerable increase of the surface through which the fluid can penetrate into the body, and the sides of the channels because of the fibrous structure with the fibres situated in planes approximately parallel with the upper and lower faces form a surface very open to penetration of fluids. The material cut or milled away during the preparation can be re-used in the dry-deposition process for which reason the forming of the channels does not cause waste of the superabsorber, which represents a substantial part of the costs of material for the preparation of the absorption body.

By the dry-deposition process the core of the absorption body as mentioned is provided with a paper layer 3. This paper layer contributes at distributing the fluid and in itself is even able to absorb part of the fluid. However, the most important function of the paper is to keep the core together during and after the milling of the channels 2. Because of the paper layer 3 the tendency of the absorption body to become squeezed together from the sides is reduced and the paper layer thereby contributes at keeping the channels open.

Below the paper 3 there may be placed a highly absorbing layer 4 of material, which layer may also be prepared as a length by dry-deposition of a mixture consisting of about 35% by weight of fluff, about 15% by weight of thermoplastic fibres and about 50% by weight of superabsorber. At a thickness of 1.5 mm this length will have a weight of about 350 g/m² in contradistinction to the core proper, which at a thickness of 5 mm will have a weight before milling of about 550 g/m². The layer 4 has the purpose of increasing the absorption capacity and it may have various shapes such as hour-glass shape or the like. In this manner it is possible to ensure an optimum capacity at the ends of the absorption body. The superabsorber is chosen with such moderate rate of absorption that the fluid will have time to spread over a substantial portion of the area of the absorption body, so as to utilize the capacity comparatively uniformly, and the absorption body better becomes able to receive a number of brief flows of urine at some intervals of time. If a particularly high absorption capacity is not needed, the highly absorbing layer 4 may be dispensed with entirely or partly.

If a particularly high absorption capacity is needed, the absorption body may be rendered both thicker and bigger. It may be rendered so much bigger that at the ends of the core it extends outside the contour thereof, whereas at the middle it is somewhat narrower than the core. According to an economic embodiment the highly absorbing layer may be replaced by or supplemented with a diaper of cellulose fluff the major parts of its volume being placed at the ends of the core. Hereby the total absorption capacity may be increased at 200-500 ml corresponding to the entire content of a full urinary bladder. In this embodiment the distributing properties of the core is utilized to a high degree whereas the absorbing capability of the core, in itself big, contributes at absorbing the entire amount of urine. Since in the area between the legs of the wearer substantially only the distributing core of the absorption body is present, this form of a diaper is considerably more comfortable than traditional diapers. At the normal absorption capacities of for instance about 70 ml, there is especially attached importance to the comfort and to the circumstance that the absorption body is to be part of a napkin which despite a big absorption capacity must be so compact that the user does not in dressing need to pay any regard to the napkin.

The core 1 and the optional highly absorbing layer 4 are thereupon wrapped in one or more layers 5 of paper of which one or more layers may have been led down to the bottom of the channels 2 to which it may be secured by the aid of glue or the like.

During the preparation of the absorption body this pressing down may take place by the aid of rollers or the like. The purpose of this or these layer(s) of paper is firstly to contribute at distributing the fluid throughout the surface of the body, instead of the fluid being possibly pressed through the core without being absorbed, and secondly to keep the pulverulent superabsorber material within the core material.

Preferably the paper is only fixed at the bottoms of the channels because this has importance for the expansion of the core and for the comfort when the absorption body is worn against the skin of the user.

On the under side the absorption body is furthermore provided with a liquid-tight layer 6 preventing seeping out of fluid.

Outermost the absorption body is enveloped in a non-woven material layer 7 which is skin-friendly and which encloses the absorption body in a closed bag.

Absorption bodies for use in cases of female incontinence are severed in lengths of about 180 mm, which severing as shown in fig. 1 may be straight but which may also be curved. In the latter case the core material and highly absorbing material 4 cut off may be re-used. At the ends 10 the envelope is closed by glueing, hot melting or the like. The finished absorption body if needed may be provided with a commonly known adhesive layer 8 at the lower face whereby the absorption body may be maintained as an ordinary napkin in the pants of the user. The adhesive layer in known manner may be covered by a releasable silicone coated cover 9.

Although the absorption body according to the invention mainly is described as a protection in cases of incontinence, for which purpose its ability to absorb very rapidly not insignificant amounts of fluid released in one or more comparatively short but comparatively strong flows is indispensable, it is within the framework of the invention to use the absorption body for absorbing other human secretions, and the shape and the size of the body may be varied according to need. As examples of such uses may be mentioned diapers, sanitary napkins and wound dressings, but even purely technical uses may come into consideration.

## Claims

1. An absorption body for use in cases of urinary incontinence in women and containing a core (1) of a highly absorbent material, said core optionally being entirely or partly surrounded by one or more layers (4) of absorbing material, wherein:

a) the core is made up in the form of a flat pad and consists substantially of a fibrous material of which at least a part constitutes a stable, three-dimensional network as a structure-giving component in which there is evenly distributed highly absorbent material;

b) the core is provided with a number of preferably rectilinear and parallel cut channels (2) on one side thereof;

characterised in that the core (1) is provided with a thin, coherent porous layer of material (3) on the side opposite said channels, said channels having a depth of 65 percent to 95 percent of the thickness of the pad, have a width of at least 4 mm, and leave at least 4 mm width of core material between each two neighbouring channels, said fibrous material comprises thermoplastic fibres or fibres coated with thermoplastic material fused together at their points of contact and fused to said thin, coherent porous layer (3) and said highly absorbent material is a superabsorbent material.

2. An absorption body according to claim 1, further characterised in that a thin, absorbing layer of material (5), preferably of porous paper, as a casing surrounds the core (1) on all sides and has been led down into the channels (2) to their bottom.

3. An absorption body according to claim 1 or claim 2 further characterised in that the fibrous material entirely or partly consists of natural fibres or other non thermoplastic fibres coated with a thermoplastic material.

4. An absorption body according to claim 1 or claim 2 characterised in that the fibrous material partly consists of cellulose fluff.

5. An absorption body according to claim 1 further characterised in that the core consists of 40 - 50% by weight of pulverulent or fibrous, cross-linked acrylic polymer, 10 - 20% by weight of thermoplastic fibres and 30 - 50% by weight of cellulose fluff.

6. An absorption body according to claim 2 or any of claims 3 to 5 as dependent on claim 2, further characterised in that the thin, absorbing layer of material (5) surrounding the core (1) on all sides and preferably consisting of paper has been secured, preferably adhesively, to the bottoms of the channels (2) and only to these.

7. An absorption body according to claim 2 or any of claims 3 to 6 as dependent on claim 2, further characterised in that a flat, highly absorbent layer (4) of fibres and fibrous or pulverulent cross-linked polymer has been placed between the core (1) and the casing (5), on the side of the core adapted to be turned away from the source of liquid, said layer having a lesser thickness than the core.

8. A method for producing an absorption body for use in cases of female urinary incontinence and containing a core (1) in the form of a flat absorbent pad comprising the steps of providing a flat absorbent pad, and forming parallel channels (2) in a major surface of the pad, characterised in that fibres, at least a proportion of which are formed of hot-meltable thermoplastic material or are coated with thermoplastic materials are dry-deposited on a thin, coherent porous sheet (3) together with pulverulent superabsorbent material, the sheet with the deposited layer of mixed fibrous material and pulverulent material is heated to a temperature sufficient to adhere the fibres together at their points of contact and to the sheet material, thereby forming three-dimensional coherent network in which the superabsorbent material is evenly distributed, and in that said channels are cut or milled to a depth of 65 percent to 95 percent of the thickness of the pad, each channel having a width of at least 4mm and the spacing between neighbouring channels being at least 4 mm.

9. A method as claimed in claim 8 wherein said temperature is in the range 90 degrees C to 200 degrees C and is just sufficient to soften the thermoplastic surface of said fibres.

10. A method as claimed in claim 8 or claim 9, further characterised in that the amount of core material removed by the cutting or milling operation is at least 25 percent of the entire volume of core material.

11. A method as claimed in claim 10, further characterised in that the amount of core material removed is 30 percent of the volume of core material.

## Patentansprüche

1. Absorptionskörper zur Verwendung bei Fällen von Harn-Inkontinenz bei Frauen, welcher einen Kern (1) aus hochabsorbierendem Material enthält und wahlweise vollständig oder teilweise von einer Schicht oder mehreren Schichten (4) aus absorbierendem Material umgeben ist, wobei:

a) der Kern in Form eines flachen Polsters zugerichtet wird und im wesentlichen aus einem faserigen Material besteht, welches zumindest teilweise ein stabiles, dreidimensionales Netzwerk als strukturbildende Komponente bildet, bei welcher hochabsorbierendes Material gleichmäßig verteilt ist;

b) der Kern mit einer Anzahl vorzugsweise geradliniger und parallel geschnittener Kanäle (2) auf seiner einen Seite versehen ist;

dadurch gekennzeichnet, daß der Kern (1) mit einer dünnen zusammenhängenden porösen Schicht aus Material (3) auf der den Kanälen gegenüberliegenden Seite versehen ist, daß die Kanäle eine Tiefe von 65 Prozent bis 95 Prozent der Dicke des Polsters aufweisen, eine Breite von zumindest 4 mm, und zumindest 4 mm Breite des Kernmaterials zwischen jeweils zwei benachbarten Kanälen stehen lassen, wobei das faserige Material thermoplastische oder mit thermoplastischem Material beschichtete Fasern aufweist, die an ihren Berührungspunkten zusammengeschmolzen sind und zu der dünnen, zusammenhängenden porösen Schicht (3) zusammengeschmolzen sind, und wobei das hochabsorbierende Material ein superabsorbierendes Material ist.

2. Absorptionskörper nach Anspruch 1, weiterhin dadurch gekennzeichnet, daß eine dünne, absorbierende Schicht aus Material (5), vorzugsweise aus porösem Papier, als ein Gehäuse den Kern (1) auf sämtlichen Seiten umgibt und in die Kanäle (2) hinein bis zu deren Boden geführt ist.

3. Absorptionskörper nach Anspruch 1 oder Anspruch 2, weiterhin dadurch gekennzeichnet, daß das faserige Material vollständig oder teilweise aus Naturfasern oder anderen nicht thermoplastischen Fasern besteht, welche mit einem thermoplastischen Material beschichtet sind.

4. Absorptionskörper nach Anspruch 1 oder Anspruch 2, dadurch gekennzeichnet, daß das faserige Material teilweise aus Zelluloseflusen besteht.

5. Absorptionskörper nach Anspruch 1, weiterhin dadurch gekennzeichnet, daß der Kern aus 40-50 Gewichtsprozent feinpulverigem oder faserigem, kreuzvernetzten Acrylpolymer besteht, 10-20 Gewichtsprozent thermoplastischen Fasern und 30-50 Gewichtsprozent Zelluloseflusen.

6. Absorptionskörper nach Anspruch 2 oder einem der Ansprüche 3 bis 5 in Rückbeziehung auf Anspruch 2, weiterhin dadurch gekennzeichnet, daß die dünne, absorbierende Schicht aus Material (5), welche den Kern (1) auf allen Seiten umgibt und vorzugsweise aus Papier besteht, an den Böden der Kanäle (2) und nur an diesen befestigt wurde, vorzugsweise durch Kleben.

7. Absorptionskörper nach Anspruch 2 oder einem der Ansprüche 3 bis 6 in Rückbeziehung auf Anspruch 2, weiterhin dadurch gekennzeichnet, daß eine flache, hochabsorbierende Schicht (4) aus Fasern und faserigem oder feinpulverigem kreuzvernetzten Polymer zwischen den Kern (1) und das Gehäuse (5) gebracht wurde, auf die Seite des Kerns, die so ausgebildet ist, daß sie von der Flüssigkeitsquelle abgewendet wird, wobei die Schicht eine geringere Dicke aufweist als der Kern.

8. Verfahren zur Herstellung eines Absorptionskörpers zur Verwendung in Fällen weiblicher Harn-Inkontinenz mit einem Kern (1) in Form eines flachen absorbierenden Polsters, mit folgenden Schritten: Bereitstellung eines flachen absorbierenden Polsters, Ausbildung paralleler Kanäle (2) in einer Hauptoberfläche des Polsters, dadurch gekennzeichnet, daß Fasern, von denen zumindest ein Teil aus heißschmelzbarem thermoplastischen Material gebildet oder mit thermoplastischem Material beschicht wird, trocken auf einem dünnen, zusammenhängenden porösen Blatt (3) zusammen mit feinpulverigem superabsorbierenden Material abgelagert werden, daß das Blatt mit der abgelagerten Schicht aus gemischtem faserigen Material und feinpulverigen Material auf eine Temperatur erhitzt wird, die dazu ausreicht, um die Fasern an ihren Berührungspunkten aneinander und mit dem Blattmaterial zu befestigen, wodurch ein dreidimensionales zusammenhängendes Netzwerk gebildet wird, in welchem das superabsorbierende Material gleichförmig verteilt ist, und daß die Kanäle geschnitten oder gerändelt werden auf eine Tiefe von 64 Prozent bis 95 Prozent der Dicke des Polsters, wobei jeder Kanal eine Breite von zumindest 4 mm aufweist und der Abstand zwischen benachbarten Kanälen zumindest 4 mm beträgt.

9. Verfahren nach Anspruch 8, bei welchem die Temperatur in dem Bereich von 90 Grad Celsius bis 200 Grad Celsius liegt und gerade ausreicht, um die thermoplastische Oberfläche der Fasern zu erweichen.

10. Verfahren nach Anspruch 8 oder Anspruch 9, weiterhin dadurch gekennzeichnet, daß die Menge des durch den Schneid- oder Rändelvorgang entfernten Kernmaterials zumindest 25 Prozent des Gesamtvolumens des Kernmaterials beträgt.

11. Verfahren nach Anspruch 10, weiterhin dadurch gekennzeichnet, daß die Menge des entfernten Kernmaterials 30 Prozent des Volumens des Kernmaterials beträgt.

**Revendications**

1. Corps d'absorption pour utilisation dans les cas d'incontinence urinaire de la femme et contenant un noyau (1) de matériau très absorbant, ledit noyau étant facultativement entièrement ou partiellement entouré par une ou plusieurs couches (4) de matériau absorbant, dans lequel:

a) le noyau est réalisé sous la forme d'un tampon plat et consiste sensiblement en un matériau fibreux dont au moins une partie constitue un réseau stable tridimensionnel en tant qu'élément constituant la structure dans lequel est distribué régulièrement du matériau très absorbant;

b) le noyau est prévu avec un nombre de canaux découpés de préférence rectilignes et parallèles (2) sur un de ses côtés;

caractérisé en ce que le noyau (1) comporte une fine couche poreuse cohérente de matériau (3) sur le côté opposé auxdits canaux, lesdits canaux ayant une profondeur de 65% à 95% de l'épaisseur du tampon, ils ont une largeur d'au moins 4 mm, et laissent au moins 4 mm de largeur de matériau du noyau entre deux canaux voisins, ledit matériau fibreux comporte des fibres thermoplastique ou des fibres revêtues de matériau thermoplastique fondu avec celles-ci en leur point de contact et fondu avec ladite couche fine poreuse cohérente (3) et ledit matériau très absorbant·est un matériau super-absorbant.

2. Corps d'absorption selon la revendication 1 caractérisé en outre en ce qu'une fine couche absorbante de matériau (5),. de préférence du papier poreux, entoure en tant que boîtier le noyau (1) sur tous ses côtés et a été déposé à l'intérieur des canaux (2) vers le fond de ces derniers.

3. Corps d'absorption selon la revendication 1 ou la revendication 2 caractérisé en outre en ce que le matériau fibreux consiste en totalité ou en partie en fibres naturelles ou en d'autres fibres non thermoplastiques revêtues d'un matériau thermoplastique.

4. Corps d'absorption selon la revendication 1 ou la revendication 2 caractérisé en ce que le matériau fibreux consiste partiellement en un duvet de cellulose.

5. Corps d'absorption selon la revendication 1 caractérisé en outre en ce que le noyau consiste en 40—50% en poids de polymère acrylique à liaisons croisées pulvérulent ou fibreux, 10 à 20% en poids de fibres thermoplastiques et 30 à 50% en poids de duvet de cellulose.

6. Corps d'absorption selon la revendication 2 ou l'une quelconque des revendications 3 à 5 selon la revendication 2, caractérisé en outre en ce que la fine couche absorbante de matériau (5) qui entoure le noyau (1) sur tous ses côtés et consistant de préférence en papier a été fixée, de préférence par collage, aux fonds des canaux (2) et seulement à ces derniers.

7. Corps d'absorption selon la revendication 2 ou l'une des revendications 3 à 6 selon la revendication 2, caractérisé en outre en ce qu'une couche plane très absorbante (4) de fibres et de polymère fibreux ou pulvérulent à liaisons croisées a été disposée entre le noyau (1) et le boîtier (5), du côté du noyau susceptible d'être tournée en éloignement par rapport à la source de liquide, ladite couche ayant une épaisseur inférieure à celle du noyau.

8. Procédé pour la fabrication d'un corps d'absorption destiné à être utilisé dans le cas d'incontinence urinaire de la femme et contenant un noyau (1) sous la forme d'un tampon absorbant plat comprenant les étapes qui consistent à prévoir un tampon absorbant plat et à former des canaux parallèles (2) dans la plus grande partie de la surface du tampon, caractérisé en ce que des fibres, au moins une partie desquelles sont réalisées en un matériau thermoplastique susceptible d'être fondu à la chaleur ou sont revêtues de matériaux thermoplastiques sont déposés à sec sur une feuille poreuse cohérente de faible épaisseur (3) avec un matériau pulvérulent super-absorbant, la feuille revêtue de la couche déposée d'un matériau fibreux mixte et d'un matériau pulvérulent est chauffee à une température suffisante pour faire adhérer les fibres les unes avec les autres en leurs points de contact et au matériau de la feuille, ce qui a pour résultat de former un réseau cohérent tridimensional dans lequel le matériau super-absorbant est réparti régulièrement et en ce que lesdits canaux sont découpés ou fraisés dans une profondeur de 65% à 95% de l'épaisseur du tampon, chaque canal ayant une largeur d'au moins 4 mm et l'espacement entre les canaux voisins étant d'au moins 4 mm.

9. Procédé tel que revendiqué dans la revendication 8 dans lequel ladite température est comprise entre 90°C et 200°C et est juste suffisante pour ramollir la surface thermoplastique desdites fibres.

10. Procédé tel que revendiqué dans la revendication 8 ou la revendication 9, caractérisé en outre en ce que la quantité de matériau du noyau éliminée par découpe ou fraisage est d'au moins 25% du volume total du matériau formant le noyau.

11. Procédé tel que revendiqué dans la revendication 10 caractérisé en outre en ce que la quantité de matériau du noyau éliminée est de 30% du volume du matériau du noyau.

Fig. 1

Fig. 2

2